(19) Europäisches Patentamt
European Patent Office
Office européen des brevets

(11) Veröffentlichungsnummer: **0 138 113**
**B1**

(12) # EUROPÄISCHE PATENTSCHRIFT

(45) Veröffentlichungstag der Patentschrift: **27.04.88**

(51) Int. Cl.⁴: **C 07 D 205/08**, C 07 D 405/04

(21) Anmeldenummer: **84111448.1**

(22) Anmeldetag: **26.09.84**

(54) **Verfahren zur Herstellung von optisch einheitlichen Azetidinonen.**

(30) Priorität: **03.10.83 CH 5365/83**

(43) Veröffentlichungstag der Anmeldung: **24.04.85 Patentblatt 85/17**

(45) Bekanntmachung des Hinweises auf die Patenterteilung: **27.04.88 Patentblatt 88/17**

(84) Benannte Vertragsstaaten: **AT BE CH DE FR GB IT LI LU NL SE**

(56) Entgegenhaltungen: **EP-A-0 073 061**

(73) Patentinhaber: **F. HOFFMANN- LA ROCHE & CO. Aktiengesellschaft, CH- 4002 Basel (CH)**

(72) Erfinder: **Schmid, Gérard, Dr., Mittler Feldweg 6, CH- 4468 Kienberg (CH)**

(74) Vertreter: **Martin, Björn, Grenzacherstrasse 124 Postfach 3255, CH- 4002 Basel (CH)**

**EP 0 138 113 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die bekannten 3,4-cis-3-Acylamino-azetidinone sind wertvolle Zwischenprodukte für die Gewinnung von antimikrobiell wirksamen β-Lactam-Antibiotika. Deren Herstellung erfolgt üblicherweise durch Cycloaddition von Phthalimido- oder Azidoacetylchlorid mit einem entsprechenden N-geschützten Imin (z. B. Benzaldehyd-(2,4-dimethoxybenzyl)imin) in Gegenwart einer Base, wonach man im erhaltenen 1-geschützten N-(2-oxo-3-azetidinyl)phthalimid bzw. -azid die Phthalimid- bzw. Azidgruppe in die Aminogruppe überführt (die Phthalimidogruppe durch Umsetzung mit Hydrazin, Methylhydrazin oder Dimethylaminopropanamin, die Azidgruppe durch Reduktion mit Ammoniumsulfid oder mit elementarem Wasserstoff und einem Katalysator, wie Palladium/Kohle) und die freigesetzte Aminogruppe aus Gründen der Weiterverarbeitung durch Umsetzen mit einem Acylchlorid, z. B. Carbobenzoxychlorid, geschützt wird.

Die direkte Cycloaddition von N-Carbobenzoxyglycinchlorid mit einem N-geschützten Imin liefert jedoch nur niedrige Ausbeuten an gewünschtem Azetidinon; vgl. J. Chem. Soc., 1880 (1975). N-Carbobenzoxyglycinchlorid selbst ist eine instabile Verbindung, die sich schon bei niedrigen Temperaturen zu unbrauchbaren Nebenprodukten zersetzt. Diese direkte Cycloaddition wurde somit als impraktikabel angesehen; vgl. Tetrahedron 37, 2321 (1980).

Es wurde nun überraschenderweise gefunden, dass eine direkte Cycloaddition mit guten Ausbeuten erzielt wird, wenn man anstelle von N-Carbobenzoxyglycinchlorid ein N-Carbobenzoxyglycin-Alkalimetallsalz verwendet und die in Gegenwart einer Base vorgenommene Cycloaddition mit dem N-geschützten Imin unter zusätzlichem Einwirken eines bestimmten Sulfonsäurechlorids, insbesondere von p-Chlorbenzolsulfonylchlorid, durchführt.

Durch Wahl eines N-geschützten Imins mit geeigneter Substitution (vgl. Formel III nachstehend) werden im Cycloadditionsprodukt (Azetidinon) zwei neue optische Zentren induziert, wobei die Substituenten in 3- und 4-Stellung cis-ständig sind und ferner mit hoher Diastereoselektivität nur eines der zwei möglichen diastereomeren Produkte gebildet wird. Die vorliegende neue Synthese führt demnach wie nachstehend gezeigt wird zu optisch einheitlichen Verfahrensprodukten.

Die vorliegende Erfindung betrifft im speziellen ein Verfahren zur Herstellung von optisch einheitlichen Azetidinonen der allgemeinen Formel

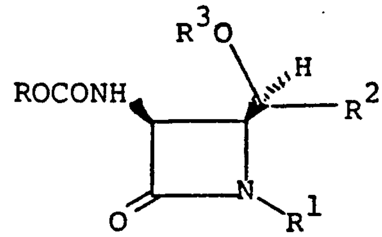

I

worin R Benzyl, β-(Trimethylsilyl)-niederes Alkyl oder R-Halogen-niederes Alkyl, $R^1$ eine leicht abspaltbare Schutzgruppe aus der Gruppe, bestehend aus Benzyl, 2,4- oder 3,4-Di(niederem Alkoxy)benzyl, Di[4-(niederem Alkoxy)phenyl]methyl, 4-(niederem Alkoxy)phenyl, niederem 2-Alkenyl und der Gruppe der Formel

$-CH_2-CH(OR^5)_2$ (a) oder

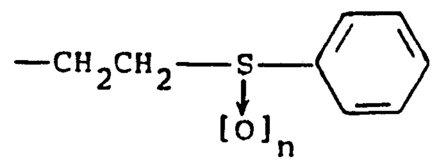

(b)

wobei $R^5$ niederes Alkyl und n die Zahl 0 oder 1 bedeuten, $R^2$ niederes Alkyl, Phenyl-niederes Alkyl oder niederes Alkoxyalkyl und $R^3$ niederes Alkyl oder Phenyl-niederes Alkyl oder $R^2$ und $R^3$ zusammen mit dem Chiralitätszentrum und dem Sauerstoffatom einen 5- oder 6-gliedrigen 0-Heterocyclus, der gegebenenfalls ein weiteres mit dem Chiralitätszentrum nicht direkt verbundenes Sauerstoffatom enthält und gegebenenfalls durch niederes Alkyl niederes Alkoxy, Oxo oder Spirocyclo-niederes Alkyl Substituiert ist, bedeuten, und die mit nieder bezeichneten Reste bis zu acht Kohlenstoffatome besitzen, das dadurch gekennzeichnet ist, dass man ein Alkalimetallsalz einer Carbonsäure der allgemeinen Formel

$ROCONHCH_2COOH$ II

worin R obige Bedeutung besitzt, mit einer optisch einheitlichen Verbindung der allgemeinen Formel

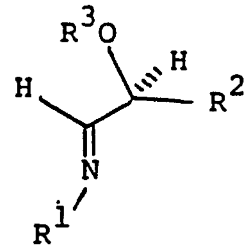

III

worin $R^1$, $R^2$ und $R^3$ obige Bedeutung besitzen, in Gegenwart einer Base und eines Sulfonsäurechlorids der allgemeinen Formel

$R^4-SO_2-Cl$ IV

worin $R^4$ Phenyl, niederes Alkylphenyl, Halogenphenyl, niederes Alkyl oder Halogen-niederes Alkyl bedeutet, umsetzt.

Bei den obigen Verfahrensprodukten der Formel I kommen als leicht abspaltbare Schutzgruppen $R^1$ die folgenden in Betracht: Benzyl, 2,4- oder 3,4-Di-(nie-deres Alkoxy)-benzyl, insbesondere 2,4- oder 3,4-Dimethoxy-benzyl, Di[4-(niederes Alkoxy)phenyl]methyl, insbesondere Di(4-methoxyphenyl)methyl, oder 4-(niederes Alkoxy)phenyl, insbesondere 4-Methoxyphenyl; ferner niederes 2-Alkenyl oder eine Gruppe der Formel

$-CH_2-CH(OR^5)_2$ (a) oder

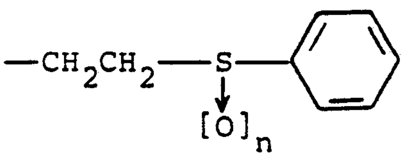 (b)

worin $R^5$ niederes Alkyl und n die Zahl 0 oder 1 bedeuten.

Der Ausdruck "leicht abspaltbare Schutzgruppe" ist im weitesten Sinne aufzufassen, schliesst also auch Gruppen ein, die an sich nicht direkt abspaltbar sind, jedoch durch einfache chemische Umwandlung in eine direkte abspaltbare Schutzgruppe übergeführt werden können. So werden z. B. folgende Gruppen $R^1$ vor der Abspaltung in die nachstehend angegebenen abspaltbaren Gruppen übergeführt:

niederes 2-Alkenyl→ niederes 1-Alkenyl $-CH_2-CH(OR^5)_2 \rightarrow -CH_2-CHO \rightarrow -CO-CH(OH)_2$

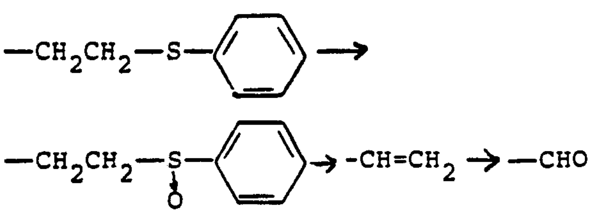

Die Umwandlung und Abspaltung dieser Gruppen werden in der Europäischen Patentpublikation Nr. 101 598 näher erläutert.

Der Ausdruck "niederes 2-Alkenyl" bezeichnet eine olefinische Kohlenwasserstoffgruppe, die geradkettig oder verzweigt sein kann, die eine Doppelbindung in 2-Stellung hat und bis zu 8, vorzugsweise bis zu 4 Kohlenstoffatome enthält, wie z. B. 2-Propenyl (Allyl), 2-Methallyl, 2-Butenyl, 2-Hexenyl, 2-Heptenyl, 2-Octenyl, usw. Der Ausdruck "niederes Alkyl" bezeichnet eine gesättigte Kohlenwasserstoffgruppe, die geradkettig oder verzweigt sein kann und bis zu 8, vorzugsweise bis zu 4 Kohlenstoffatome enthält, wie z. B. Methyl, Aethyl, n-Propyl, Isopropyl, n-Butyl, Isobutyl, sec-Butyl, t-Butyl, n-Pentyl, Isopentyl, n-Hexyl, n-Heptyl, n-Octyl, usw.

Der Ausdruck "niederes Alkoxy" bezeichnet eine über ein Sauerstoffatom gebundene niedere Alkylgruppe.

Bevorzugte Gruppen $R^1$ sind: 2-Propenyl (Allyl), 2,2-Dimethoxyäthyl, 2,2-Diäthoxyäthyl, 2-Phenylthioäthyl, 2-Phenylsulfinyläthyl, 2,4- oder 3,4-Dimethoxybenzyl und Benzyl. Allyl, Benzyl und 2,4-Dimethoxybenzyl sind die am stärksten bevorzugten Bedeutungsmöglichkeiten von $R^1$.

Bevorzugte Gruppen

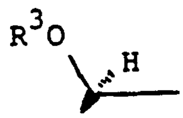 si:

sind solche, worin $R^2$ niederes Alkyl, Phenyl-niederes Alkyl, niederes Alkoxyalkyl, z. B. niederes Alkoxymethyl und $R^3$ niederes Alkyl oder Phenyl-niederes Alkyl bedeuten. Die Gruppen

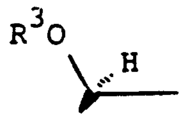 können

können auch einen 5- oder 6-gliedrigen 0-Heterocyclus darstellen, der gegebenenfalls ein weiteres mit dem Chiralitätszentrum nicht direkt verbundenes Sauerstoffatom enthält und gegebenenfalls durch niederes Alkyl, niederes Alkoxy, Oxo oder Spirocyclo-niederes Alkyl substituiert sein kann. Beispiele für solche Gruppen, welche ebenfalls bevorzugt werden, sind:

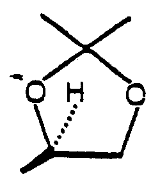, 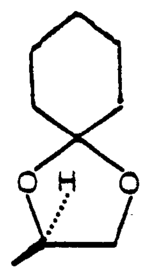, 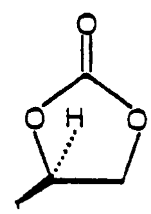,

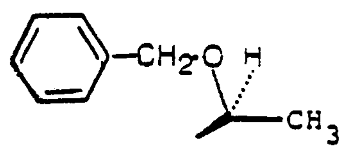

Besonders bevorzugt wird die Gruppe

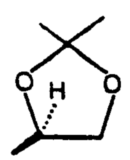 [(R)-2,2--

[(R)-2,2- Dimethyl-1,3-dioxolan-4-yl].

Bei den Verfahrensprodukten der Formel I ist R vorzugsweise Benzyl. R kann aber, wie bereits erwähnt, auch β-(Trimethylsilyl)-niederes Alkyl, z. B. β-(Trimethylsilyl)-äthyl, oder β-Halogen-niederes Alkyl, z. B. β-Trichloräthyl, β-Dichloräthyl, β-Chloräthyl oder β-Trichlorisopropyl, bedeuten.

Als Alkalimetallsalze einer Carbonsäure der Formel II kommen die Kalium-, Natrium- und auch die Lithiumsalze in Frage. Bevorzugt werden die Kaliumsalze.

Als Sulfonsäurechlorid der Formel IV wird vorzugsweise das p-Chlorbenzolsulfonsäurechlorid verwendet. Für die Zwecke der vorliegenden Erfindung eignen sich aber auch das p-Toluolsulfonsäurechlorid und das Methansulfonsäurechlorid.

Die Umsetzung der Verbindungen der Formel II, III und IV erfolgt in Gegenwart einer Base, beispielsweise eines tertiären Amins, wie Triäthylamin, und in einem inerten organischen Lösungsmittel, in wasserfreier Form, wobei vorzugsweise Äther, wie Tetrahydrofuran, Diäthyläther, t-Butylmethyläther, Dioxan, Äthylenglykoldimethyläther oder dergleichen, halogenierte Kohlenwasserstoffe, wie Methylenchlorid, Chloroform, 1,2-Di-chloräthan oder dergleichen, Acetonitril, Dimethylformamid oder dergleichen, in Frage kommen. Die Temperatur der Umsetzung liegt im Bereiche von etwa -30° bis etwa 50° C.

Die Überführung einer Verbindung der Formel I in ein antimikrobiell wertvolles β-Lactam-Antibiotikum wird z. B. in der Europäischen Patentpublikation Nr. 73061 und in der Europäischen Patentpublikation Nr. 101 598 beschrieben. Die dabei notwendige Abspaltung von $R^1$ in der Bedeutung "Benzyl" erfolgt in der gleichen Weise wie für $R^1$ als "2,4- bzw. 3,4-Di(niederes Alkoxy)benzyl", d.h. oxidativ mit Hilfe eines gepufferten Peroxodisulfats, wie Kaliumperoxodisulfat/Dikaliumhydrogensulfat. Die Benzylgruppe kann aber auch reduktiv durch Einwirken eines Alkalimetalls, z. B. von Natrium oder Lithium, in flüssigem Ammoniak abgespalten werden.

**Beispiel 1**

30 g wasserfreies Magnesiumsulfat und 17,7 g (71,6 mMol) N-Carbobenzoxyglycin-Kaliumsalz werden in 400 ml Methylenchlorid dispergiert. Nach Zusatz von 20 ml (143 mMol) Triäthylamin wird die erhaltene Suspension bei Zimmertemperatur 1 1/2 Stunden heftig gerührt und anschliessend auf 5° C abgekühlt. Diese Suspension wird mit 10,0 g (35,8 mMol) Isopropyliden-D-glyceraldehyd-(2,4-dimethoxybenzyl)-imin (hergestellt aus 2,4-Dimethoxybenzylamin und Isopropyliden-L-glyceraldehyd in 20 ml Methylenchlorid) versetzt. Anschliessend werden 15,1 g (71,6 mMol) p-Chlorbenzolsulfonylchlorid in 50 ml Methylenchlorid innert 45 Minuten bei 5° C zugetropft. Die Suspension wird 3 Stunden bei Zimmertemperatur gerührt und filtriert. Das Filtrat wird eingedampft, das erhaltene gelbbraune Öl in 300 ml Äthylacetat gelöst und nacheinander zweimal mit 100 ml IN wässriger Salzsäure, zweimal mit 100 ml 5 %-iger wässriger Natriumbicarbonatlösung und einmal mit 100 ml wässriger Kochsalzlösung gewaschen. Die organische Phase wird eingedampft und der ölige gelbe Rückstand durch Zugabe von 300 ml Äther bei 0° C kristallisiert. Man erhält 10,5 g (62 %) Benzyl (3S,4S)-cis-1-(2,4-dimethoxybenzyl)-4-[(R)- 2,2-dimethyl-1,3-dioxolan-4-yl]-2-oxo-3-azetidincarbamat vom Schmelzpunkt 113-114° C.

**Beispiel 2**

Falls in Beispiel 1 anstelle von 2,4-Dimethoxybenzylamin Allylamin eingesetzt wird, erhält man in der gleichen Weise, nach Chromatographierung an Kieselgel (0,040 bis 0,063 mm), 7,05 g (67 %) Benzyl (3S,4S)-cis-1-Allyl-4-[(R)- 2,2-dimethyl-1,3-dioxolan-4-yl]-2-oxo-3-azetidincarbamat vom Schmelzpunkt 94-96° C.

**Patentansprüche**

1. Verfahren zur Herstellung von optisch einheitlichen Azetidinonen der allgemeinen Formel

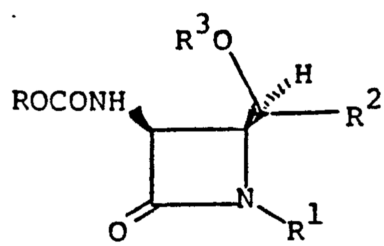

I

worin R Benzyl, β-(Trimethylsilyl)-niederes Alkyl oder β-Halogen-niederes Alkyl, $R^1$ eine leicht abspaltbare Schutzgruppe aus der Gruppe, bestehend aus Benzyl, 2,4- oder 3,4-Di(niederem Alkoxy)benzyl, Di[4-(niederem Alkoxy)phenyl]methyl, 4-(niederem Alkoxy)phenyl, niederem 2-Alkenyl und der Gruppe der Formel

$-CH_2-CH(OR^5)_2$ (a) oder

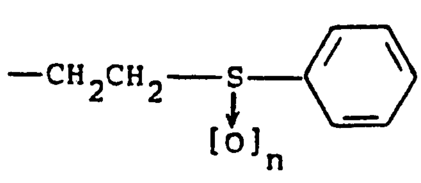

(b)

wobei $R^5$ niederes Alkyl und n die Zahl 0 oder 1 bedeuten, $R^2$ niederes Alkyl, Phenyl-niederes Alkyl oder niederes Alkoxyalkyl und $R^3$ niederes Alkyl oder Phenyl-niederes Alkyl oder $R^2$ und $R^3$ zusammen mit dem Chiralitätszentrum und dem Sauerstoffatom einen 5- oder 6-gliedrigen O-Heterocyclus, der gegebenenfalls ein weiteres mit dem Chiralitätszentrum nicht direkt

verbundenes Sauerstoffatom enthält und gegebenenfalls durch niederes Alkyl niederes Alkoxy, Oxo oder Spirocyclo-niederes Alkyl substituiert ist, bedeuten, und die mit nieder bezeichneten Reste bis zu acht Kohlenstoffatome besitzen,

dadurch gekennzeichnet, dass man ein Alkalimetallsalz einer Carbonsäure der allgemeinen Formel

$ROCONHCH_2COOH$ II

worin R obige Bedeutung besitzt, mit einer optisch einheitlichen Verbindung der allgemeinen Formel

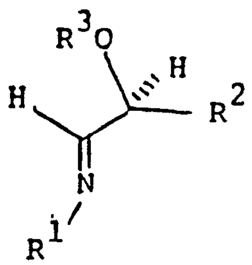

III

worin $R^1$, $R^2$ und $R^3$ obige Bedeutung besitzen, in Gegenwart einer Base und eines Sulfonsäurechlorids der allgemeinen Formel

$R^4$-$SO_2$-Cl IV

worin $R^4$ Phenyl, niederes Alkylphenyl, Halogenphenyl, niederes Alkyl oder Halogen-niederes Alkyl bedeutet, umsetzt, wobei die Umsetzung in einem wasserfreien inerten organischen Lösungsmittel bei einer Temperatur im Bereich von etwa -30° C bis etwa 50° C durchgeführt wird.

2. Verfahren nach Anspruch 1, dadurch gekennzeichnet, dass man ein Alkalimetallsalz einer Carbonsäure der Formel II verwendet, worin R Benzyl bedeutet.

3. Verfahren nach Anspruch 1 oder 2, dadurch gekennzeichnet, dass man ein Kaliumsalz einer Carbonsäure der Formel II verwendet.

4. Verfahren nach einem der Ansprüche 1-3, dadurch gekennzeichnet, dass man eine Verbindung der Formel III verwendet, worin die Gruppe

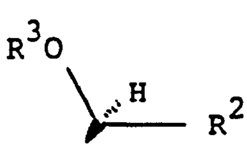

die Gruppe

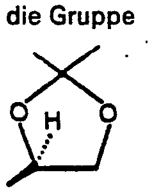

bedeutet.

5. Verfahren nach einem der Ansprüche 1-4, dadurch gekennzeichnet, dass man eine Verbindung der Formel III verwendet, worin $R^1$ Allyl, Benzyl oder 2,4-Dimethoxy-benzyl bedeutet.

6. Verfahren nach einem der Ansprüche 1-5, dadurch gekennzeichnet, dass man eine Verbindung der Formel IV verwendet, worin $R^4$ p-Chlorphenyl bedeutet.

7. Verfahren nach einem der Ansprüche 1-6, dadurch gekennzeichnet, dass man als Base Triäthylamin verwendet.

8. Verfahren nach einem der Ansprüche 1-7, dadurch gekennzeichnet, dass man die Reaktion in wasserfreiem Methylenchlorid, durchführt.

**Claims**

1. A process for the manufacture of optically uniform azetidinones of the general formula

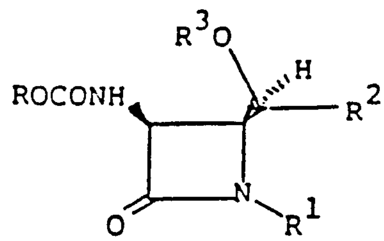

I

wherein R signifies benzyl, β-(trimethylsilyl)-lower alkyl or β-halo-lower alkyl, $R^1$ signifies a readily cleavable protecting group from the group consisting of benzyl, 2,4- or 3,4-di(lower alkoxy)benzyl, di[4-(lower alkoxy)phenyl]methyl, 4-(lower alkoxy)phenyl, lower 2-alkenyl and the group of the formula

$-CH_2-CH(OR^5)_2$ (a) or

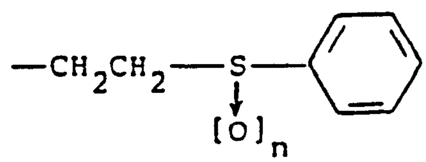

(b)

in which $R^5$ signifies lower alkyl and n signifies the number 0 or 1, $R^2$ signifies lower alkyl, phenyl-lower alkyl or lower alkoxyalkyl and $R^3$ signifies lower alkyl or phenyl-lower alkyl or $R^2$ and $R^3$ together with the centre of chirality and the oxygen atom signify a 5 or 6-membered O-heterocycle which optionally contains a further oxygen atom not directly linked with the centre of chirality and which is optionally substituted by lower alkyl, lower alkoxy, oxo or spirocyclo-lower alkyl, and the residues denoted by lower have up to eight carbon atoms,

characterized by reacting an alkali metal salt of a carboxylic acid of the general formula

$ROCONHCH_2COOH$ II

wherein R has the above significance, with an

optically uniform compound of the general formula

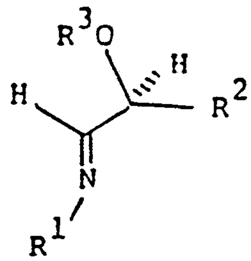

III

wherein $R^1$, $R^2$ and $R^3$ have the above significance, in the presence of a base and a sulphonic acid chloride of the general formula

$R^4-SO_2-Cl$ IV

wherein $R^4$ signifies phenyl, lower alkylphenyl, halophenyl, lower alkyl or halo-lower alkyl, whereby the reaction is carried out in an anhydrous inert organic solvent at a temperature in the range of about -30° C to about 50° C.

2. A process according to claim 1, characterized in that an alkali metal salt of a carboxylic acid of formula II in which R signifies benzyl is used.

3. A process according to claim 1 or 2, characterized in that a potassium salt of a carboxylic acid of formula II is used.

4. A process according to any one of claims 1-3, characterized in that a compound of formula III in which the group

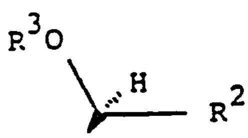

$R^2$ signifies the group

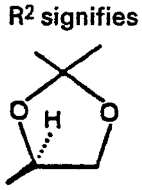

is used.

5. A process according to any one of claims 1-4, characterized in that a compound of formula III in which $R^1$ signifies allyl, benzyl or 2,4-dimethoxybenzyl is used.

6. A process according to any one of claims 1-5, characterized in that a compound of formula IV in which $R^4$ signifies p-chlorophenyl is used.

7. A process according to any one of claims 1-6, characterized in that triethylamine is used as the base.

8. A process according to any one of claims 1-7, characterized in that the reaction is carried out in anhydrous methylene chloride.

**Revendications**

1. Procédé de préparation d'azétidinones optiquement purs de formule générale

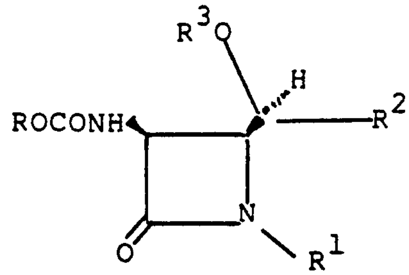

I

dans laquelle R représente un groupe benzyle, bêta-(triméthylsilyl)-alkyle inférieur ou bêta-halogéno-alkyle inférieur, $R^1$ représente un groupe protecteur facile à éliminer choisi parmi les groupes benzyle, 2,4- ou 3,4-di-(alcoxy inférieur)benzyle, di-[4-(alcoxy inférieur)phényl]-méthyle, 4-(alcoxy inférieur)phényle, 2-alcényle inférieur et le groupe de formule

$-CH_2-CH(OR^5)_2$ (a) ou

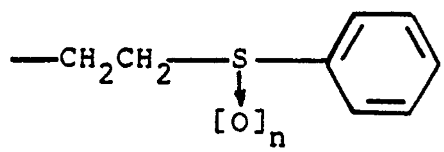

(b)

$R^5$ représentant un groupe alkyle inférieur et n étant égal à 0 ou 1, $R^2$ représente un groupe alkyle inférieur, phényl-alkyle inférieur ou alcoxyalkyle inférieur et $R^3$ représente un groupe alkyle inférieur ou phényl-alkyle inférieur ou bien $R^2$ et $R^3$ forment ensemble et avec le centre de chiralité et l'atome d'oxygène, un hétérocycle oxygéné à 5 ou 6 chaînons qui peut contenir, le cas échéant, un autre atome d'oxygène non relié directement au centre de chiralité et est, le cas échéant, substitué par un groupe alkyle inférieur, alcoxy inférieur, oxo ou spirocyclo-alkyle inferieur, les groupes qualifiés d'"inférieurs" contenant jusqu'à 8 atomes de carbone, caractérisé en ce que l'on fait réagir un sel de métal alcalin d'un acide carboxylique de formule générale

$ROCONHCH_2COOH$ II

dans laquelle R a les significations indiquées ci-dessus, avec un composé optiquement pur de formule générale

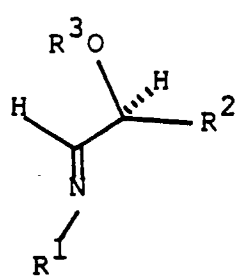

III

dans laquelle $R^1$, $R^2$ et $R^3$ ont les significations indiquées ci-dessus, en présence d'une base et d'un chlorure d'acide sulfonique de formule générale

$R^4-SO_2-Cl$ IV

dans laquelle $R^4$ représente un groupe phényle, (alkyle inférieur)-phényle, halogénophényle, alkyle inférieur ou halogéno-alkyle inférieur, la réaction étant effectuée dans un solvant organique inerte anhydre à une température dans l'intervalle de -30 à 50°C environ.

2. Procédé selon la revendication 1, caractérisé en ce que l'on utilise un sel de métal alcalin d'un acide carboxylique de formule II dans laquelle R représente un groupe benzyle.

3. Procédé selon la revendication 1 ou 2, caractérisé en ce que l'on utilise un sel de potassium d'un acide carboxylique de formule II.

4. Procédé selon l'une des revendications 1 à 3, caractérisé en ce que l'on utilise un composé de formule III dans laquelle le groupe $R^3$

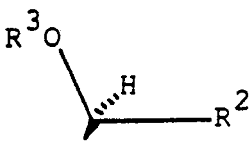

est le groupe

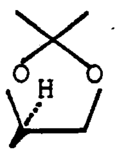

5. Procédé selon l'une des revendications 1 à 4, caractérisé en ce que l'on utilise un composé de formule III dans laquelle $R^1$ représente un groupe allyle, benzyle ou 2,4-diméthoxybenzyle.

6. Procédé selon l'une des revendications 1 à 5, caractérisé en ce que l'on utilise un composé de formule IV dans laquelle $R^4$ représente un groupe p-chlorophényle.

7. Procédé selon l'une des revendications 1 à 6, caractérisé en ce que l'on utilise en tant que base la triéthylamine.

8. Procédé selon l'une des revendications 1 à 7, caractérisé en ce que l'on effectue la réaction dans du chlorure de méthylène anhydre.